# EUROPEAN PATENT SPECIFICATION

(11) **EP 1 826 273 B1**
(45) Date of publication and mention of the grant of the patent: **15.10.2014**
(21) Application number: 07003607.4
(22) Date of filing: 21.02.2007
(51) Int. Cl.: C12N 15/90, C12R 1/66

(54) **Method for the production of a strain having a deleted region in chromosome**
Verfahren für die Herstellung eines Stammes mit deletierter Chromosomenregion
Procédé pour la production d'une souche ayant une région supprimée au niveau du chromosome

(30) Priority: 22.02.2006 JP 2006045895
(43) Date of publication of application: 29.08.2007
(73) Proprietor: NODA INSTITUTE FOR SCIENTIFIC RESEARCH, Noda-shi Chiba 278-0037 (JP)
(72) Inventor: Takahashi, Tadashi, Noda-shi, Chiba 278-0037 (JP); Koyama, Yasuji, Noda-shi, Chiba 278-0037 (JP)
(74) Representative: Strehl Schübel-Hopf & Partner

(56) References cited:
- EP-A- 1 666 601
- WO-A-01/68882
- US-A1- 2003 199 037
- TADASHI TAKAHASHI ET AL: "Enhanced gene targeting frequency in ku70 and ku80 disruption mutants of Aspergillus sojae and Aspergillus oryzae" MOLECULAR GENETICS AND GENOMICS ;, SPRINGER-VERLAG, BE, vol. 275, no. 5, 10 February 2006 (2006-02-10), pages 460-470, XP019428009 ISSN: 1617-4623
- TAKAHASHI TADASHI ET AL: "Identification and analysis of Ku70 and Ku80 homologs in the koji molds Aspergillus sojae and Aspergillus oryzae" BIOSCIENCE BIOTECHNOLOGY AND BIOCHEMISTRY, vol. 70, no. 1, January 2006 (2006-01), pages 135-143, XP002434049 ISSN: 0916-8451
- KOOISTRA ROLF ET AL: "Efficient gene targeting in Kluyveromyces lactis" YEAST, CHICHESTER, SUSSEX, GB, vol. 21, no. 9, 15 July 2004 (2004-07-15), pages 781-792, XP002371803 ISSN: 0749-503X
- NINOMIYA Y ET AL: "Highly efficient gene replacements in Neurospora strains deficient for nonhomologous end-joining" PROCEEDINGS OF THE NATIONAL ACADEMY OF SCIENCES OF USA, NATIONAL ACADEMY OF SCIENCE, WASHINGTON, DC, US, vol. 101, no. 33, 17 August 2004 (2004-08-17), pages 12248-12253, XP002346025 ISSN: 0027-8424
- KRAPPMANN S ET AL: "GENE TARGETING IN ASPERGILLUS FUMOGATUS BY HOMOLOGOUS RECOMBINATION IS FACILITATED IN A NONHOMOLOGOUS END- JOINING-DEFICIENT GENETIC BACKGROUND" EUKARYOTIC CELL, AMERICAN SOCIETY FOR MICROBIOLOGY, US, vol. 5, no. 1, January 2006 (2006-01), pages 212-215, XP009063272 ISSN: 1535-9778
- DA SILVA FERREIRA M E ET AL: "THE AKUB(KU80) MUTANT DEFICIENT FOR NONHOMOLOGOUS END JOINING IS A POWERFUL TOOL FOR ANALYZING PATHOGENICITY IN ASPERGILLUS FUMIGATUS" EUKARYOTIC CELL, AMERICAN SOCIETY FOR MICROBIOLOGY, US, vol. 5, no. 1, January 2006 (2006-01), pages 207-211, XP009063271 ISSN: 1535-9778
- TAKAHASHI T ET AL: "Efficient gene disruption in the koji-mold Aspergillus sojae using a novel variation of the positive-negative method" MGG - MOLECULAR GENETICS AND GENOMICS, SPRINGER, BERLIN, DE, vol. 272, no. 3, October 2004 (2004-10), pages 344-352, XP002371805 ISSN: 1617-4615
- TAKAHASHI TADASHI ET AL: "Nonfunctionality of Aspergillus sojae aflR in a strain of Aspergillus parasiticus with a disrupted aflR gene" APPLIED AND ENVIRONMENTAL MICROBIOLOGY, vol. 68, no. 8, August 2002 (2002-08), pages 3737-3743, XP002434050 ISSN: 0099-2240
- DATABASE EMBL [Online] 6 January 2006 (2006-01-06), "Aspergillus oryzae DNA, aflatoxin biosynthesis gene cluster, complete sequence, strain: RIB40." XP002434052 retrieved from EBI accession no. EMBL:AB196490 Database accession no. AB196490

## Description

### Technical Field

The present invention relates to a method for the production of a strain having a deleted region in chromosome (a deletant) derived from a mitosporic filamentous fungus belonging to *Aspergillus sojae* or *Aspergillus oryzae,* and to the strain having a deleted region in chromosome thus obtained.

### Background of the Invention

*Aspergillus* strains such as *Aspergillus sojae* and *Aspergillus oryzae* have been industrially used in the production of brewed food such as soy sauce, sake (rice wine), soybean paste, etc. With a recent determination of the whole genomic sequence of *Aspergillus oryzae* and development of an exhaustive analysis of gene expression using a micro-array, it has been expected that genetic modification of their genes, especially their chromosomal modification would increase the productivity of an enzyme and improve a growth rate of these filamentous fungi.

However, as *Aspergillus* strains have a very low frequency of homologous recombination, it has been very difficult to produce a strain with a large area-deletion of an arbitrary region in chromosome by conventional methods. For example, as the frequency of homologous recombination is as low as 1~3 %, it will be necessary to prepare several tens to hundreds of transformants and select a desired strain from them in order to integrate even a single vector into an optional area of its chromosome (Non-Patent Document 1). Furthermore, it is necessary to select a recombinant strain having a recombination between both ends of an area to be deleted for obtaining the strain with a large-area deletion. But, it would be very difficult to select such recombinant for *Aspergillus* strains that have the very low frequency of homologous recombination. However, a recent study by the present inventors has revealed that the disruption of a gene involved in non-homologous recombination will significantly increase the frequency of gene targeting (homologous recombination).

By the way, Aflatoxin is a toxin produced by fungi belonging to *Aspergillus* such as *Aspergillus flavus* or *Aspergillus parasiticus* and is known as the strongest cancer-causing agent among natural substances. There have been big problems about contamination of cereal due to these molds in tropical region. There was death en mass of turkeys in England in 1960, which was caused by uptake of the cereal contaminated by the mold (an accident of turkey's X disease). Since the above *Aspergillus* strains are closely-related to Aflatoxin-producing strains, they have been studied in a wide variety of views with suspicion of their Aflatoxin-producing capability (Non-Patent Document 2). As a result, it has been confirmed that although they will not produce Aflatoxin under any conditions, they have a homologue of a gene cluster of about 60 Kb for the Aflatoxin-biosynthesis of comprised the Aflatoxin-producing strains (Non-Patent Document 3).

It has been therefore strongly desired that the gene cluster should be removed from the *Aspergillus* strains for a further increase of safety of foods. However, it has been very difficult to prepare a mutant strain having deletion of the above Aflatoxin cluster since a set of genes in this cluster are not expressed (Non-Patent Document 4), making it impossible to carry out selection based on its phenotype in addition to the low frequency of their homologous recombination.
[Non-Patent Document 1] Takahashi et al., Mol. Gen. Genet. (2004) 272:344-52
[Non-Patent Document 2] Matsushima et al., Journal of the Japan Soy Sauce Research Institute, (2004) 31:5-11
[Non-Patent Document 3] Yu et al. (2004) Appl Environ Microbiol 70:1253-1262
[Non-Patent Document4] Matsushima et al., Appl Microbiol Biothechnol 55: 771-776
[Non-Patent Document 5] Hirashima et al. (2006) Nucleic Acids Res 34:e11

Tadashi Takahashi et al.:"Enhanced gene targeting frequency in ku 70 and ku80 disruption mutants of Aspergillus sojae and Aspergillus oryzae", MOLECULAR GENETICS AND GENOMICS, Springer Verlag, BE, vol. 275, no. 5, 10 February 2006, pages 460-470 discloses an increased gene targeting frequency in ku70 and ku80 disruption mutants of A. sojae and A. oryzae.

Tadashi Takahashi et al.:"Identification and analysis of ku70 and ku80 homologs in the koji molds Aspergillus sojae and Aspergillus oryzae", BIOSCIENCE BIOTECHNOLOGY AND BIOCHEMISTRY, vol. 70, no. 1, January 2006, pages 135-143 characterizes ku70 and ku80 in A. sojae and A. oryzae and discloses that homologous recombination is highly increased in ku disrupted mutants of A. sojae and A. oryzae.

### Disclosure of the Invention

The main purpose of the present invention is therefore to provide a transformant which will not produce a toxic substance such as Aflatoxin even after being manipulated with genetic engineering by efficiently deleting a large chromosomal region with a length of from several tens to hundreds kb such as a cluster of biosynthesis genes encoding the toxic substances such as Aflatoxin.

The present inventors succeeded in efficiently producing a strain having a deleted region in chromosome with such a large length as from several tens to hundreds kb by using *Aspergillus* with an increased frequency of homologous recombination, and completed the present invention.

Thus, the present invention is related to a method for the production of a strain having a deleted region in chromosome (deletant) using a transformant having an increased frequency of homologous recombination, comprising transforming said transformant so as to include a homologous region in both ends of a chromosomal region to be deleted, and deleting the chromosomal region by means of homologous recombination based on said homologous region.

The present invention provides the following.
[1] A method for the production of a strain having a deleted region in chromosome using a transformant derived from *Aspergillus sojae* or *Aspergillus oryzae* and having an increased frequency of homologous recombination due to disruption of ku70 and/or ku80, comprising transforming said transformant so as to include a homologous region in both ends of a chromosomal region to be deleted, and deleting the chromosomal region by means of homologous recombination between said homologous region, wherein the homologous region is a DNA sequence originally existing in the same chromosome as that of the chromosomal region to be deleted and the homologous region consists of a DNA sequence comprising a gene or its part which is located at the most peripheral site in either end of the chromosomal region to be deleted, wherein the method further comprises amplifying a DNA sequence comprising a gene or its part which is located at the most peripheral site in one end of the chromosomal region to be deleted, and integrating the amplified sequence into a gene located at the most peripheral site in the other end of said chromosomal region as a homologous region by homologous recombination so that the homologous regions will be included in both ends of said chromosomal region.
[2] A method according to [1] above, wherein the chromosomal region constitutes a cluster of genes involved in biosynthesis of a toxic substance.
[3] A method according to [2] above, wherein the toxic substance is Aflatoxin.
[4] A method according to [1] above, wherein the homologous region is a DNA sequence comprising moxY gene, and is integrated into pksA gene.
[5] A method according to [4] above, wherein the homologous region has a DNA sequence represented by SEQ ID No.7.
[6] A method according to any one of [1] to [6] above, wherein a marker gene used for negative selection is comprised in the chromosomal region to be deleted.
[7] A method according to [6] above, wherein the marker gene used for negative selection is selected from pyrG, sC or niaD.

The transformant used in the present method is a mitosporic filamentous fungus belonging to Aspergillus sojae or Aspergillus oryzae having the increased frequency of homologous recombination due to disruption of a *Ku gene,* which can be prepared in accordance with the method described in the present specification.

According to the present invention, it is possible to efficiently delete the large chromosomal region with a length of from several tens to hundreds kb such as a cluster of Aflatoxin-biosynthesis genes. As a result, it is easy to produce a strain having deletion of a gene cluster occupying a large area in the chromosome, which is very preferable from safety point of view.

The same method for deletion is known for yeasts (Non-Patent Document 5). However, the yeast is well known as originally having a high frequency of homologous recombination, for example, about 88% frequency of homologous recombination with a homologous region of about 600bp. On the other hand, there has been no report about deletion of a large region for the *Aspergillus* strains that has the low frequency of homologous recombination. It has now become possible for the first time to produce a deletant of a large region in chromosome only by using the *Aspergillus* strains having the increased frequency of homologous recombination

### Brief Description of Drawings

Fig.1 is a schematic figure showing a process of the production of a *Ku70* gene-disruption strain.
Fig.2 is a schematic figure showing a process of the production of a *Ku70* antisense RNA-expressing strain.
Fig.3 is a schematic figure showing a process of the production of a *Ku80* gene-disruption strain.
Fig.4 is a photograph of electrophoresis showing the results of disruption of *Ku70* of *Aspergillus sojae.*
Fig.5 is a photograph of electrophoresis showing the results of insertion of *ptrA* into the *Ku70* site of *Aspergillus sojae.*
Fig.6 is a schematic figure showing a process of the production of a tannase gene-disruption strain.
Fig.7 is a photograph showing the results of screening of the tannase gene-disruption strain by their phenotypes. An arrow shows strains wherein the tannase gene is not disrupted.
Fig.8 is a photograph of electrophoresis showing the results of disruption of tannase gene.
Fig.9 is a photograph of electrophoresis showing the results of disruption of *Ku80.*
Fig.10 is a photograph of electrophoresis showing the results of disruption of *Ku70* of *Aspergillus oryzae.*
Fig.11 is a schematic view of strategy for the production of a strain wherein the cluster of Aflatoxin (AF) genes was deleted.
Fig.12 is a photograph of southern blot showing the structure of a vector for deletion of the cluster of Aflatoxin genes and the results of its integration.

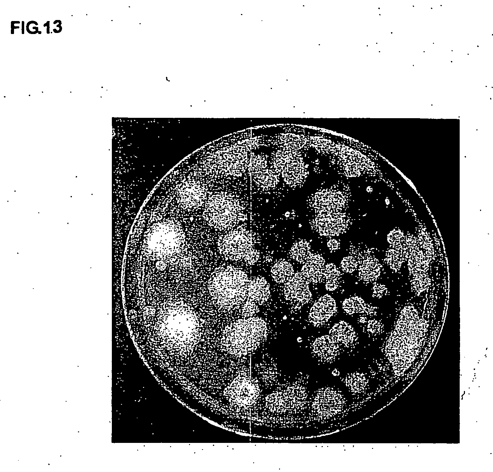

Fig.13 is a photograph showing the preparation of a 5FOA-resistant strain.
Fig.14 is a photograph of southern blot showing the production of *Aspergillus sojae* strain wherein the cluster of Aflatoxin genes was deleted.
Fig.15 shows markers that can be used for both positive- and negative-selection of *Aspergillus* strains.

### Best Mode for Carrying Out the Present Invention

There is no limitation on size, location in the chromosome, etc. of the chromosomal region to be deleted. It is, however, one of the features of the present invention that a large chromosomal region with a length of from several tens to hundreds kb can be deleted. A preferable example of such chromosomal region is the cluster of genes involved in biosynthesis of substances such as Aflatoxin, which should preferably not be produced by the strain from a safety aspect. The size and location in the chromosome of said genes are known for those skilled in the art. For example, the cluster of genes involved in biosynthesis of Aflatoxin of *Aspergillus sojae* comprises 23 genes and accounts for about 60 kb.

There is no limitation on the origin, DNA sequence, length, etc. of the homologous region (double-stranded DNA) as long as the chromosomal region wedged between the homologous regions can be deleted by means of homologous recombination. The homologous region may be a sequence derived from a different strain from that to be transformed or artificially manufactured by DNA synthesis.

The length of the homologous region should be normally more than several hundreds bp, preferable more than 1kb in order to efficiently conduct homologous recombination.

In case the homologous regions are sequenced in a reverse direction with each other, excision may not occur well or the chromosome may be possibly interrupted. Both homologous regions should therefore be preferably sequenced in the same direction.

The homologous region is a DNA sequence that originally exists in the same chromosome as that of the chromosomal region to be deleted. When such DNA sequence is used, a transformant wherein the homologous regions are included in both ends of said chromosomal region will be produced by amplifying the DNA sequence with any DNA amplification technique known for those skilled in the art such as PCR using the chromosome as a template, and integrating the amplified sequence into the other end of said chromosomal region by homologous recombination as a homologous region. The above homologous recombination for integration of the homologous region may be carried out by amplifying a base sequence comprising the sequence at a site in the other end of said chromosomal region into which the homologous region will be integrated by means of an appropriate DNA amplification technique such as PCR using the chromosome as a template, and inserting the homologous region into the thus amplified sequence with a restriction enzyme to obtain a "vector for deletion."

When the chromosomal region to be deleted is the cluster of genes, the transformant wherein the homologous regions are included in both ends of said chromosomal region will be easily produced by amplifying a DNA sequence comprising a gene or its part which located at the most peripheral site in one end of the chromosomal region to be deleted by means of an appropriate DNA amplification technique such as PCR, and using the amplified sequence as a homologous region.

Accordingly, if the chromosomal region to be deleted is the cluster of Aflatoxin genes, a DNA sequence comprising moxY gene will be used as the homologous region and integrated into pksA gene as described in Example 6 of the present specification so as to easily produce the desired transformant.

As the DNA sequence comprising a gene or its part which is located in both ends of the cluster of the genes is included in the homologous region in such case, it will remain in the chromosome after the target chromosomal region has been deleted. However, since the other genes in said cluster have been deleted, the substance encoded by said cluster of the genes shall not be biosynthesized any more. As a result, the purpose of the present invention, i.e., the provision of a strain that is very preferable from safety point of view, shall be fully attained. Accordingly, it is not necessary for the cluster of genes to be deleted to include all of the genes involved in biosynthesis of a substance.

Alternatively, the advantages of the present invention can be effected as long as a part of the DNA sequence of the gene located in both ends of the cluster has been deleted so that the function of the gene such as transcription or translation of a functional protein or peptides is not maintained any more. Accordingly, it is not necessary to completely delete the gene located in both ends of the cluster of genes to be deleted.

It is further preferable that a marker gene used for negative selection known for those skilled in the art is comprised in the chromosomal region to be deleted in order to efficiently select a strain having the chromosomal-region deletion after having been produced by transforming said transformant so as to include the homologous region in both ends of the chromosomal region to be deteted, and deleting the chromosomal region by homologous recombination between said homologous regions. There is no limitation on the site into which the marker gene is inserted. It is preferable to insert it into the vector for deletion in advance by a treatment with a restriction enzyme and the like.

As a result, transformants whose chromosomal region has not been deleted will lead to death due to conversion of a selectable drug into a cytotoxic substance by an expression product of the marker gene during the culture of the transformants in a medium comprising the selection drug. On the other hand, the chromosomal-region deletion transformant can grow in the above medium so as to be selected.

Furthermore, in order to confirm that the homologous region has been integrated into the other end of said chromosomal region to be deleted, it is preferable to insert a maker gene for positive selection into the vector for deletion as well.

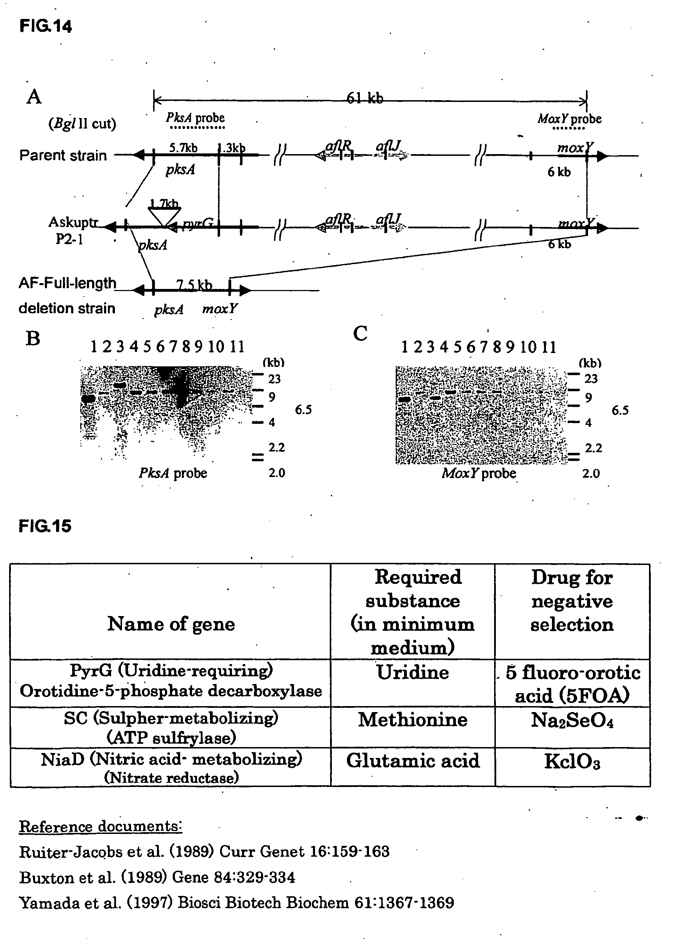

Those maker genes for negative and positive selection may be the same or different with each other. A marker gene that can be used for both selections is preferable, including pyrG, sC and niaD as shown in Fig.15.

The "transformant having an increased frequency of homologous recombination" is prepared by disrupting *Ku gene* of *A. sojae* or *A. oryzae.*

As already described in the above, the *Ku gene* such as *Ku70* and *Ku80* is a gene involved in the non-homologous recombination mechanism. Its representative examples include *Ku70* gene (SEQ ID NO.1) and *Ku80* gene (SEQ ID NO.2 or SEQ ID NO.3) derived from *Aspergillus sojae,* and *Ku70* gene (SEQ ID NO.4) and *Ku80* gene (SEQ ID NO.5 or SEQ ID NO.6) derived from *Aspergillus oryzae.* Homology (identity) in an amino acid level between the above *Ku70* genes and between the above *Ku80* genes was found to be as high as 95% or more. On the other hand, the homology in an amino acid level between these genes and homologues of *Neurospora crassa* is about 50%.

Accordingly, there may be mentioned as preferable examples of the *Ku gene,* a gene that encodes a protein consisting of an amino acid sequence represented by any one of SEQ ID Nos 1-6, or a protein consisting of the same amino acid sequence wherein one or several amino acid residues are replaced, deleted, or added, and having the function relating to non-homologous recombination mechanism.

The above protein may have a high homology such as about 80% or more, preferably about 90% or more, more preferably about 95% or more on a total average to the amino acid sequence represented by any one of SEQ ID Nos 1-6. The homology between the amino acid sequences may be determined by means of algorithm known to those skilled in the art such as BLAST.

Furthermore, preferable examples of the *Ku gene* may include (a) DNA comprising a coding region represented by any one of SEQ ID Nos 1-6, or DNA being hybridized with DNA consisting a DNA sequence complementary with that of the DNA (a) under stringent conditions, and encoding a protein having a function relating to non-homologous recombination mechanism.

The coding region in any one of SEQ ID Nos 1-6 was determined based on the information about the genomic sequences of *Aspergillus sojae* and *Aspergillus oryzae*, comparison with the sequences of their homologues of *Neurospora crassa* and rules concerning an intron sequence such as GT-AG rule. The genomic sequence of the *Ku gene* of *Aspergillus sojae* was determined by amplifying a fragment in PCR using a genomic DNA of *Aspergillus sojae* ATCC46250 as a template and primers kuU459-KuL4222 and ku2U830 and kuL4937 (prepared based on the genomic sequences of *Aspergillus oryzae* and their homologues of *Neurospora crassa*), cloning the resulting fragment by means of TOPO-TA cloning kit (Invitrogen Co.) and subjecting it to a conventional sequence determination.

The hybridization may be performed in accordance with a method known in the art, for example, that described in Current protocols in molecular biology (edited by Frederick M. Ausubel et al., 1987). When a commercially available library is used, the hybridization may be done according to instructions attached to it.

The term "stringent conditions" means in this specification, for example, those of sodium concentration of 150~900mM, preferably 600~900mM, pH of 6-8 at 60°C~68°C.

The DNA that is hybridized with DNA consisting of a DNA sequence complementary with that of the DNA comprising a coding region represented by any one of SEQ ID Nos 1-6 may include, for example, DNA consisting of a DNA sequence having identity (homology) with the whole DNA sequence of said DNA, of about 90% or more, preferably of about 95% or more on a total average. The identity between the DNA sequences may be determined by means of algorithm known to those skilled in the art, such as BLAST

The disruption of the *Ku gene* in the transformant according to the present invention may be performed by any method known to those skilled in the art such as those actually described in the examples of the present specification. For example, the *Ku gene* may be disrupted by means of a *Ku gene*-disruption vector. The resulting transformant may have the frequency of homologous recombination that is increased by at least 10 times, preferably by at least 60 times.

The transformant produced in Example 1, Δ *Ku70* strain (ASKUPTR8) derived from *Aspergillus sojae* I-6 strain (wh, *ΔpyrG*), was deposited at the International Patent Organism Depository of National Institute of Advanced Industrial Science and Technology at AIST Tsukuba Central 6, 1-1, Higashi 1- chome Tsukuba-shi, Ibaraki-ken 305-8566 Japan on December 2, 2004 with Accession No. FERM P-20311, and then transferred to international deposit under Budapest Treaty on November 17, 2005 with Accession No. FERM BP-10453.

The present invention will be specifically explained below with reference to the examples, which should not be construed to limit the scope of the present invention. Unless otherwise described, the means and conditions for gene engineering techniques in the following examples are usual ones known to those skilled in the art, such as those described in Japanese Patent Publication No.1998-80196.

PCR may be carried out in accordance with conditions and means known to those skilled in the art by using a primer set for amplification according to the present invention. For example, the PCR is done by heating for 2 min at 94°C, followed by repeating 30 times a cycle of heating for 10 seconds at 94°C, for 20 seconds at 55°C, and for 2 min. at 72°C, and finally by for 5 min at 72°C. A usual thermal cycler such as a "9600" model manufactured by Perkin Elmer Co. may be used. A commercially available thermal resistance DNA polymerase such as ExTaq DNA polymerase (TAKARA SHUZO CO., LTD.) may be used, and the composition of a reaction mixture may be adjusted in accordance with manufacturer's instructions attached to the polymerase product. The primers used in the PCR according to the present invention are summarized in Table 3.

### [Production of a strain having an increased frequency of homologous recombination]

### Strains:

*Aspergillus sojae* I-6 strain (wh, Δ *pyrG*) and *Aspergillus oryzae* RIB40Δ *pyrG* strain were used. The *Aspergillus sojae* I-6 strain was a *pyrG* deletion strain (Takahashi et al.2004) prepared from ATCC46250, and the *Aspergillus oryzae* RIB40 Δ *pyrG* strain was a *pyrG* deletion strain prepared from *Aspergillus oryzae* ATCC42149.

### Culture medium:

Polypeptone dextrin (PD) medium (polypepton 1%, dextrin 2%, KH₂PO₄ 0.5%, NaNO₃ 0.1 %, MgSO₄ 0.05%, casamino acid 0.1 %, pH 6.0) , CzapekDox (CZ) minimum medium, and 1.2M sorbitol CZ (as regeneration medium) were used. CZ medium containing 2mg/ml 5fluoroortic acid (SIGMA) and 20mM Uridine was used for positive selection of a pyrG-strain. KClO₃-mono-methylammonium-CZ agar plates (470 mM KClO₃, 100 mM mono-methylammonium, CZ) was used for negative selection of an *areA C* end-disruption strain. Tannic acid medium (Glucose 1%, Tannic-acid 1%, NH₄PO₄ 0.2%, KH₂PO₄ 0.2%, MgSO₄ 0.1%, Agar 1.5%, pH 7.5) was used for selection of Tannnase-disruption strain.

### Transformation:

Conidium was inoculated on liquid PD medium (50 ml) containing 20 mM Uridine in a conical flask (150 ml) and subjected to shake culture for about 20 hours at 30°C, followed by collection of mycelium. The collected mycelium was washed with 0.7M KCI buffer, shaken gently in 0.7M KCI buffer containing 1% Lysing enzyme (Sigma Co.) for 3 hours at 30°C to prepare protoplast. The protoplast was washed with 1.2M sorbitol buffer, and tranformed by means of a protoplast PEG method. Regeneration of the resulting transformant was carried out on 1.2M sorbitol CZ medium containing 0.5% agar.

### Construction of Ku70-disruption vector:

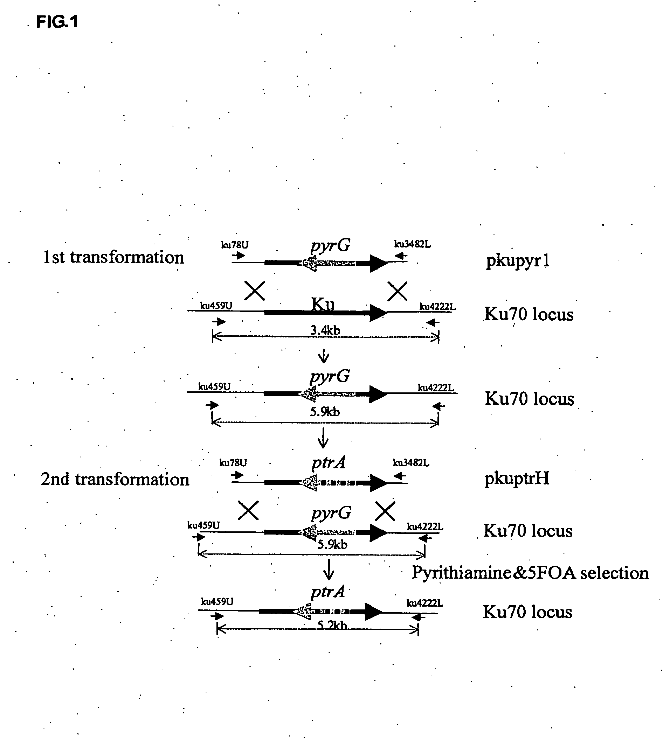

PCR was performed using the genomic DNA of *Aspergillus sojae* as a template, and primers ku78U and ku3482L (prepared based on the *Ku70* sequence of *Neurospora crassa* and its corresponding genomic sequence of *Aspergillus* oryzae). The base sequence of an amplified DNA fragment with 3.4kb was determined to confirm that the resulting fragment comprised the *Ku70* gene. This fragment was cloned by means of TOPO-TA cloning kit (Invitrogen Co.). A *ku* fragment was excised from the resulting plasmid with EcoRI and sub-cloned into pUC18. The resulting pUC18 plasmid was cut with *Bgl*II, and ligated with a 2.7kb DNA fragment containing *pyrG*, which had been amplified using primers pyrU204Bg and pyrL2924Bg having a *Bgl*II site at their ends. This ligation gave a vector pkupyr1 wherein a 600 bp region within the *Ku70* had been replaced by *pyrG* (Fig.1). A 2.1 kb *ptrA* (pyrithiamine resistant gene) fragment having *Bgl*II end sites and *ptrA* that had been amplified using pPTRI (TAKARA SHUZO CO., LTD.) as a template and primers ptrBg2482U and ptrBg4443L was ligated with the pkupyr1 cut with *Bgl*II to give a vector pkuptrH wherein said 600 bp region had been replaced by *ptrA* (Fig.1).

### Construction of Ku70 antisense RNA-expressing vector:

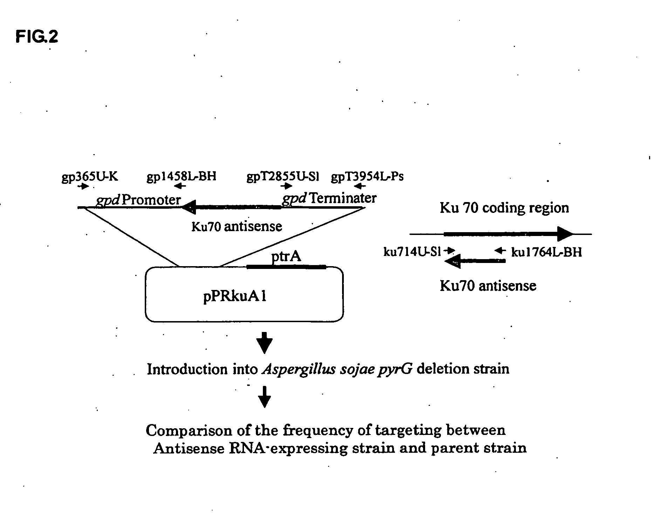

Sequences of a promoter and a terminator of *gpdA* (glycelaldehyde 3-phosphate dehydrogenase gene) were obtained from the genomic DNA of *Aspergillus sojae,* to which a 5'-end half of the coding region of the *Ku70* was ligated in a reverse direction to give a vector expressing *Ku70* antisense RNA (Fig.2). Thus, the *gpd* promoter was amplified using primers gp365U-K and gp1458L-BH, cloned by means of TOPO-TA cloning kit, and sub-cloned into a *Kpn*I*-Bam*HI site of pUC18. Said Ku fragment was amplified using primers ku714U-S1 and ku1764L-BH, cloned by means of TOPO-TA cloning kit and inserted into a *Bam*HI-*Sal*I site in the same vector. Finally, the *gpd* terminator amplified with use of primers gpT2855U-S1 and gpT3954L-Ps was inserted into a *Sal*I-*Pst*I site to give a construct for the expression of the Ku antisense RNA. A 3kb Kpnl-Pstl fragment containing the resulting construct was inserted into a *Kpn*I*-Pst*I site of pPTR1 to give a *Ku70* antisense RNA-expressing vector, pPRkuA1.

### Construction of Ku80-disruption vector:

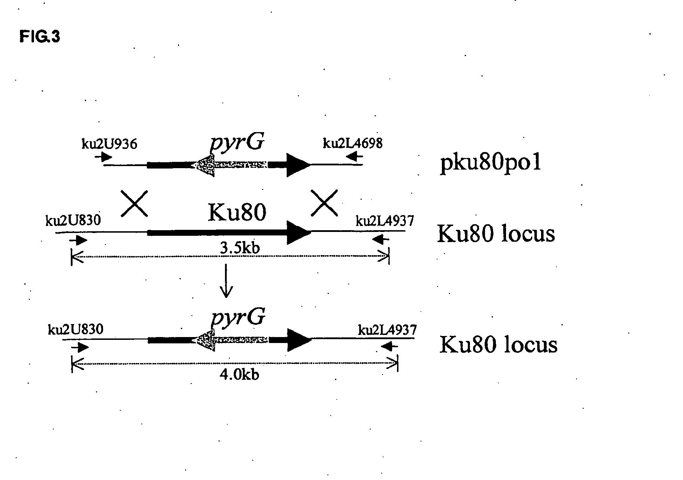

PCR was performed using the genomic DNA of *Aspergillus sojae* as a template, and primers ku2U936Xb and ku2L4698K (prepared based on the *Ku80* sequence of *Neurospora crassa* and its corresponding genomic sequence of *Aspergillus oryzae*). The base sequence of an amplified DNA fragment with 3.9kb was determined to confirm that the resulting fragment comprised the *Ku80* gene. This fragment was cloned by means of TOPO-TA cloning kit (Invitrogen Co.). The resulting vector was cut with *Bgl*II*-Mun*I *a*nd ligated with a 2.7kb DNA fragment containing *pyrG,* which had been amplified using primers pyrU204Bg and pyrL2923E, to give a *Ku80*-disruption vector, pKu80pol (Fig.3).

### [Example 1]

A *Ku70*-disruption strain was prepared and the effect of *Ku70*-disruption on the frequency of targeting (homologous recombination) was examined.

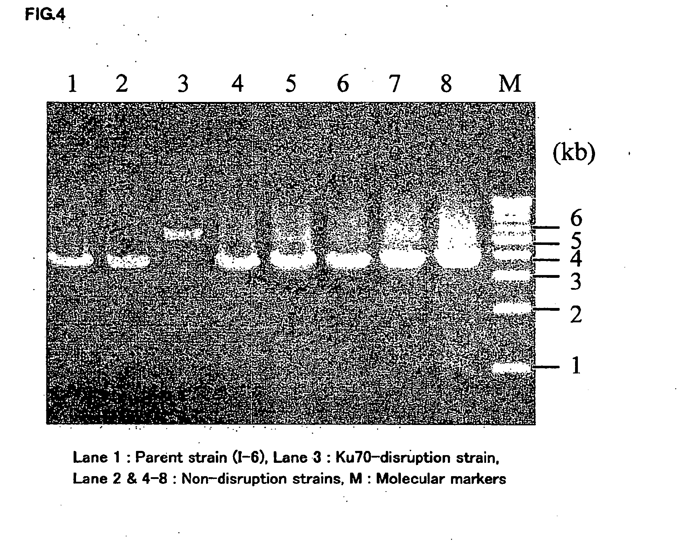

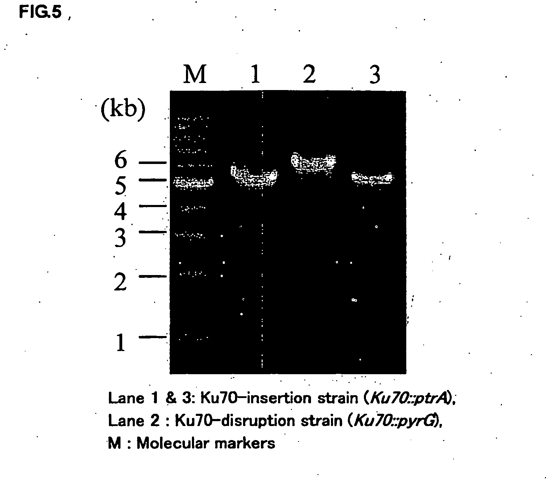

Thus, PCR was performed using said *Ku70-*disruption vector pkupyr1 as a template, and primers ku78U and ku3482L to amplify a DNA fragment, which was then used for transformation of the *pyrG* deletion strain of *Aspergillus sojae.* Genomic DNA was obtained from the transformant regenerated on 1.2M sorbitol CZ medium containing 0.5% agar, and subjected to PCR using primers kuU459-kuL4222. As a result, out of 99 transformants was obtained one *Ku gene*-disruption strain that had an amplified fragment with a length shifted from 3.4kb to 5.9kb (Fig.4). The resulting one strain was then transformed in a conventional way with a fragment obtained by amplification of the vector pkuptrH wherein *pyrG* had been replaced by *ptrA* with use of primers ku78U-3482L. After the resulting transformants were transferred to 2mg/ml-5FOA-CZ, 5FOA-resistant strains were selected. Genomic DNA was extracted from the selected strains and subjected to PCR using primers kuU459-kuL4222. As a result, it was confirmed that 5 strains out of 12 transformants had an amplified fragment with a length shifted from 5.9kb to 5.2kb, showing that the same 5 strains were *pyrG* deletion one wherein *pyrG* within *AsKu70* had been replaced by *ptrA.* The transformant according to the present invention was named *Aspergillus sojae* ASKUPTR8 strain (Fig.5). No remarkable phenotypes were observed for these strains, and there was no difference in growth rate and reduction of spore-attaching property between these strains and their parent strain.

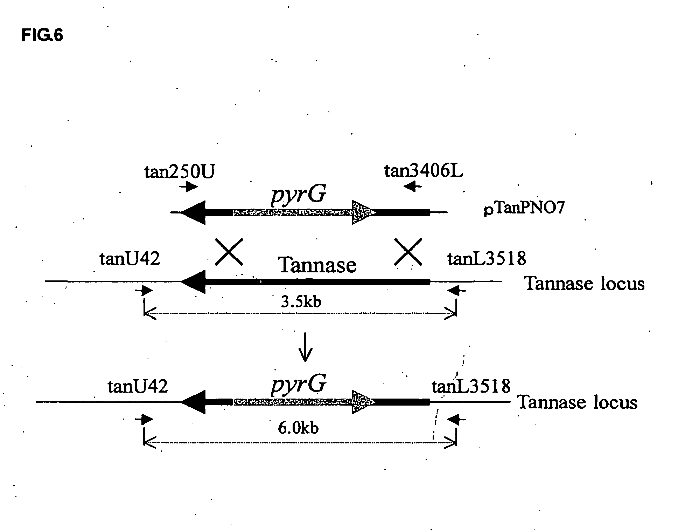

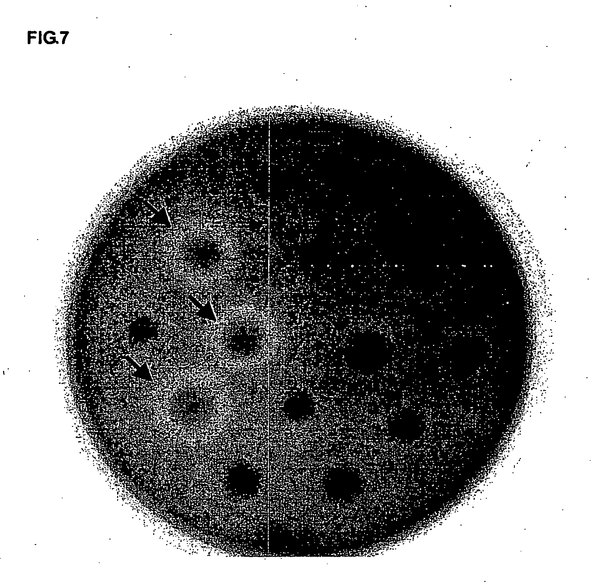

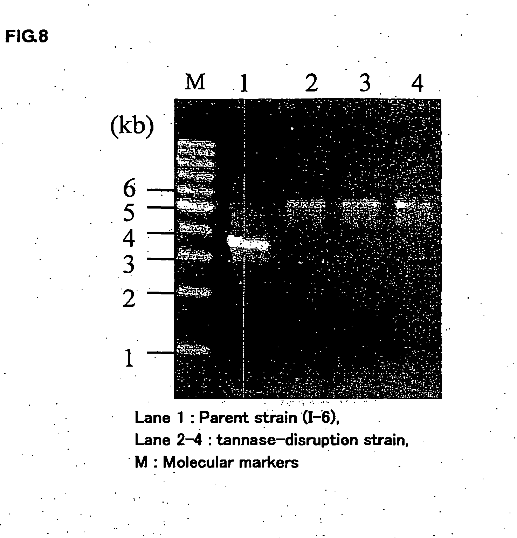

ΔKu70 strain (ASKUPTR8) was then used to obtain a tannase gene-disruption strain by homologous recombination, and the frequency of homologous recombination (gene disruption) was studied. A tannase gene-disruption vector, pTanPNO7 was used (Takahashi et al., 2004 Mol. Gen Genet.). PCR was done using pTanPNO7 as a template and primers tanU250Xb-tanL3406EI to amplify a fragment for gene disruption. *Aspergillus sojae pyrG* deletion strain I-6 and *Ku70*-disruption strain ASKUPTR8 were transformed with the resulting fragment (Fig.6). A wild-type strain of *Aspergillus sojae* would form halo on a tannic acid plate. On the other hand, as the tannase gene-disruption strain would not from the halo, it could be easily screened (Fig.7). Accordingly, each resulting transformant was inoculated on the tannic acid plate to observe whether the halo would be formed or not. Most of the transformants derived from the parent I-6 strain formed the halo. Table 1 showed that only 2 tannase gene-disruption strains were obtained among 150 strains with disruption frequency of about 1.3%. On the other hand, 42 transformant strains out of 56 strains derived from the *Ku70*-disruption strain ASKUPTR8 did not form the halo, showing a remarkable increase in disruption frequency up to 75% (Table 1A). Furthermore, the disruption of the tannase gene was confirmed by a shift in the length of a fragment from 3.5kb to 6.0Kb, which was obtained by amplification using the genomic DNA of the transformant as a template and primers tanU42-tanL3518 (Fig.8). An arm length of homologous region in the above homologous recombination was 1.4kb.

### [Example 2]

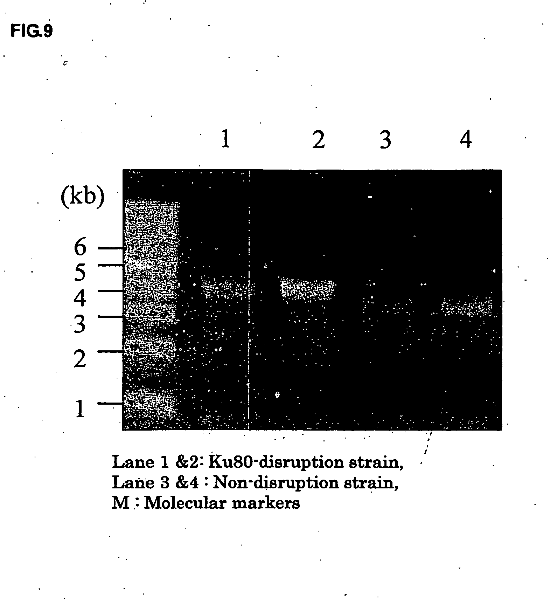

The frequency of gene disruption with respect to the *Ku80* gene was then examined using the Δ *Ku70* strain. PCR was done using the *Ku80*-disruption vector pKu80pol (Fig.3) as a template and primers ku2U936Xb-ku2L4698K to amplify a fragment for gene disruption. *Aspergillus sojae pyrG* deletion strain I-6 and the *Ku70-*disruption strain ASKUPTR8 were transformed with the resulting fragment. PCR was done using the genomic DNA extracted from the transformant as a template and primers ku2U830-ku2L4937. As a 3.5kb band in the parent strain shifted to 4.0kb band in the gene-disruption strain, the disruption strain could be easily identified (Fig.9). It was confirmed that the *Ku80* gene was disrupted in only one strain out of 42 I-6 strains with disruption frequency of 2.4%, and in 18 strains out of 25 *Ku70*-disruption (ASKUPTR8) strains with an increased disruption frequency of 72% (Table 1 (C)). An arm length of homologous region in the above homologous recombination was 1.0Kb.

### [Example 3]

A *Ku70* antisense RNA-expressing strain was prepared and its effect on the frequency of homologous recombination was examined.

Thus, the *Ku70* antisense RNA-expressing vector, pRkuA1 in a circular state was introduced into *Aspergillus sojae pyrG* deletion strain I-6 to obtain the transformants (kuA1, kuA2, kuA3 and kuA4) according to the present invention. Selection of the transformants was done with prythiamine. The introduction of the construct for expressing the *Ku70* antisense RNA was confirmed by PCR and Southern hybridization. Disruption experiment of areA's C-end was carried out using these four strains and a vector arePXB (Takahashi et al., 2004). The four strains were transformed with arePXB vector cut with *Not*l*-Xho,* and resulting transformants were transferred to KClO3-mono-methylammonium(100 mM)-CZ medium. The number of the strains with suppression of growth was counted. As a result, while the disruption of areA's C-end was observed at a ratio of about 0 or 0.7 % in the transformants derived from the parent I-6 strain, the same disruption was observed at ratio of about 12.5% and 8% in the kuA1, kuA3 and kuA4 strains showing an increase by 10 times or more (Table 1 (B)). An arm length of homologous region in the above homologous recombination was 0.9kb.

Disruption experiment of tannase was carried out using these four strains and the vector pTanPNO7 (Takahashi et al., 2004). A wild-type strain of *Aspergillus sojae* would form halo on the tannic acid plate. On the other hand, as the tannase gene-disruption strain would not form the halo, it could be easily screened. As a result, while the disruption of tannase was observed at a ratio of about 1 % in the transformants derived from the parent I-6 strain, the same disruption was observed at ratio of about 16% and 12% in the kuA1 and kuA4 strains, respectively, showing an increase by 10 times or more (Table 1A).

### [Example 4]

The effect of *Ku70*-disruption in *Aspergillus oryzae* on the frequency of tannase-disruption was examined.

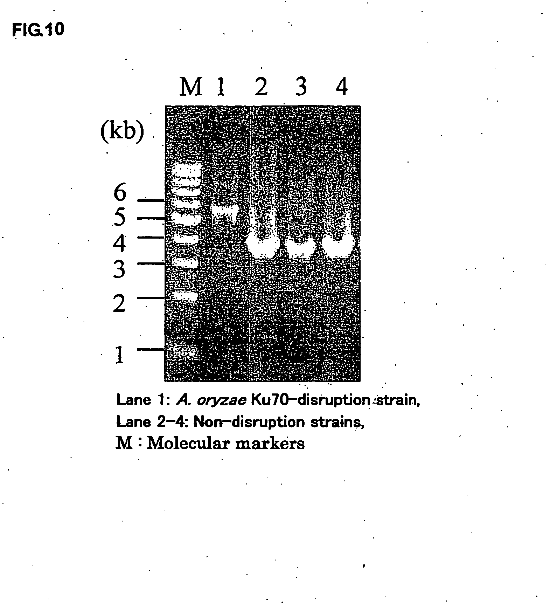

Thus, protoplast prepared from and *Aspergillus oryzae* RIB40 *pyrG* deletion strain was transformed in a conventional method with a DNA fragment amplified by PCR using the *Ku70*-disruption vector pkupyr1 as a template, and primers ku78U and ku3482L. Genomic DNA was obtained from the transformant regenerated on 1.2M sorbitol CZ medium, and subjected to PCR using primers kuU459-kuL4222. As a result, out of 30 transformants was obtained three *Ku gene*-disruption strains that had an amplified fragment with a length shifted from 3.4kb to 5.9kb (Fig.10). The resulting three strains were then transformed in a conventional way with a fragment obtained by amplification of the vector pkuptrH wherein 2.7kb *Bgl*II fragment containing *pyrG* had been replaced by *ptrA* with use of primers ku78U-3482L. After the resulting transformants were transferred to 2mg/ml-5FOA-CZ, 5FOA-resistant strains were selected. Genomic DNA was extracted from the selected strains and subjected to PCR using primers kuU459-kuL4222. As a result, it was confirmed that 4 strains out of 6 transformants had an amplified fragment with a length shifted from 5.9kb to 5.2kb, showing that the same 4 strains were *pyrG* deletion one wherein *pyrG* within *AsKu70* had been replaced by *ptrA.* The transformant according to the present invention was named *Aspergillus oryzae* strain RkuN16ptr1.

In order to study the frequency of tannase gene-disruption, a fragment for gene disruption was prepared by PCR using pTanPNO7 (Takahashi et al., 2004 Mol. Gen Genet.) as a template and primers tan U250Xb-tanL3406 El. *Aspergillus orzae Ku70*-disruption strain RkuN16ptr1 was transformed with the resulting fragment. The resulting 22 transformants were inoculated on the tannic acid plate to observe whether the halo would be formed or not. As a result, 14 transformant strains out of the above 22 strains did not form the halo, showing a remarkable increase in disruption frequency up to 63.4% (Table 1(A)).

### [Example 5]

The effect of an arm length in a homologous region on the targeting frequency was examined using the tannase gene locus as a target.

Thus, fragments having the arm length of 500bp, 100bp and 50bp were amplified by PCR using the tannase-disruption vector as a template and primers tanU889-tanL2473, tanU1350-tanL2134, and tanU1379-tanL1986, respectively, and used for transformation of the *Ku70*-disruption strain (ASKUPTR8). The results in Table 2 show that each targeting frequency were 14.3%, 0% and 0% in the targeting with use of the fragments with the arm length of 500bp, 100bp and 50bp, respectively. These results demonstrate a positive correlation between the arm length in a homologous region and the frequency of homologous recombination. Accordingly, if the arm length in a homologous region is as short as 100bp or less, little homologous recombination strain can be obtained. On the other hand, even if the arm length in a homologous region is about 500bp, the targeting can be performed with a frequency of about 14% (Table 2).

**[Table 1]**

| (A) Disruption of tannase gene | | | | | |
|---|---|---|---|---|---|
| Parent Strain | I-6 (*ΔpyrG*) | kuA1 | kuA4 | ASKUPTR8 | RkuN16ptr1 |
| The Number of Transformant | 150 | 18 | 25 | 56 | 22 |
| The Number of Disruption Strain | 2 | 3 | 3 | 42 | 14 |
| Frequency of Targeting (%) | 1.3 | 16 | 12 | 75 | 63.4 |
| l-6: *pyrG* deletion strain, kuA1 & kuA4: *Ku70* antisense RNA-expressing strain | | | | | |
| ASKUPTR8: A. *sojae Ku70*-disruption strain, | | | | | |
| RkuN16ptr1: *A*. *oryzae Ku70*-disruption strain | | | | | |
| | | | | | |

| (B) Disruption of | areA gene | | | | |
|---|---|---|---|---|---|
| Parent Strain | I-6 (Δ *pyrG*) | I-6 (Δ *pyrG*) | kuA1 | kuA3 | kuA4 |
| The Number of Transformant | 295 | 24 | 24 | 24 | 24 |
| The Number of Disruption Strain | 2 | 0 | 3 | 2 | 2 |
| Frequency of Targeting (%) | 0.7 | 0 | 12.5 | 8 | 8 |
| l-6: *pyrG* deletion strain, kuA1, kuA3 & kuA4: *Ku70* antisense RNA-expressing strain, ASKUPTR8: A. *sojae Ku70*-disruption strain, | | | | | |

| (C) Disruption of *Ku80* gene | | | | | |
|---|---|---|---|---|---|
| Parent Strain | I-6 (Δ*pyrG*) | ASKUPTR8 | | | |
| The Number of Transformant | 42 | 25 | | | |
| The Number of Disruption Strain | 1 | 18 | | | |
| Frequency of Targeting (%) | 2.4 | 72 | | | |
| I-6: *pyrG* deletion strain, ASKUPTR8: *A. sojae Ku70*-disruption strain | | | | | |

**[Table 2]**

| Parent Strain | I*-*6 (Δ*pyrG*) | ASKUPTR8 | | | |
|---|---|---|---|---|---|
| Arm Length of Homologous region (bp) | 1400 | 1400 | 500 | 100 | 50 |
| The Number of Transformant | 150 | 56 | 14 | 28 | 35 |
| The Number of Disruption Strain | 2 | 42 | 2 | 0 | 0 |
| Frequency of Targeting (%) | 1.3 | 75 | 14.3 | 0 | 0 |
| I-6: *pyrG* deletion strain, ASKUPTR8: *A. sojae Ku*70*-*disruption strain | | | | | |

**Table 3**

| Primer | Sequence |
|---|---|
| gp365U-K | GGTACCCCAGTACAGTTTCATGCAAAGTTCTA |
| gp 1458L-BH | GGATCCTTGGGGGTAGCCATTGTTTAGATGTGT |
| gpT2855U-Sl | GTCGACGGCCAGTAGGAATCAGGACAGAG |
| gpT3954L-Ps | CTGCAGCCAAGCCTGTCGTCTTGGGCTATTACG |
| ku78U | TTGCACATTTCCTGGCATTGGTATTCGG |
| kuU459 | AAATGCGACAGCACGTCCTCCCTTCC |
| ku714U-Sl | GTCGACGGATGAGTTGGAGCTGAAGCGAATGG |
| ku1764L-BH | GGATCCTAATTGCTGTTAGCAGCGATACTTCA. |
| ku3482L | ACATAGACGAGGACCAAAAGTCCCTACAG |
| kuL4222 | GGCGTTGTTAGAGGGCTTTCGTCCGTTT |
| ku2U830 | CGGTGGCTTTGGTTCGAGAGGTACGA |
| ku2U936Xb | GTGGTCTAGAATGCTCGGCATGTCTGCGGTAT |
| ku2L4698K | ACAGGGTACCCCGTAAAATCGATATTGGAAAG |
| ku2L4937 | AGAGGCAGACGATGGAAGATCAGGACCA |
| pyrU204Bg | AGATCTGGTAATGTGCCCCAGGCTTGTCAG |
| pyrL2924Bg | AGATCTTTTCCCACAGGTTGGTGCTAGTC |
| pyrL2939E | GGGGAATTCCGCGGCCTTTACCAAGGTATCGCGA |
| ptr-Bg2482U | AGATCTCATTGGTAACGAAATGTAAAAGCTA |
| ptr-Bg4443L | AGATCTGGGGTGACGATGAGCCGCTCTTGGATC |
| tanU42 | GGAACCTGGACATTCTCACTCCTCGCGT |
| tanU250Xb | TCTAGACAGCCACGAAGGTTTTGCCTTT |
| tanU889 | TGGATAGCTTTGCACGCGCAAGGGTC |
| tanU1350 | TGCTTTGGCAGCAGGAGCGAACGCAG |
| tanU1379 | CTTTTACCGATGTGTGCACCGTGTCT |
| tanL1986 | GAAAGCCGGGGCACCAGTAATCGCAC |
| tanL2134 | CAACACCGTCGGTTCTTCCATCAAGCGG |
| tanL2473 | GGATGTTGAGCTCCCACTTGCCAGTGTC |
| tanL3406EI | GAATTCTGTTGGTGGGCTTTTGCGTGTGGT |
| tanL3518 | CGAGACGGTCCAGGTCCAGGTCTAGGTCTG |

### [Example 6]

### Production of a strain having a deleted region in chromosome

### Method:

*Aspergillus sojae* ASKUPTR8 strain (wh, ΔpyrG, Ku70::ptrA) produced in Example 1 was used. Culture medium and transformation method were the same as those in the production of the transformant having an increased frequency of homologous recombination.

### Construction of a vector for deletion of a cluster of Aflatoxin-biosynthesis genes:

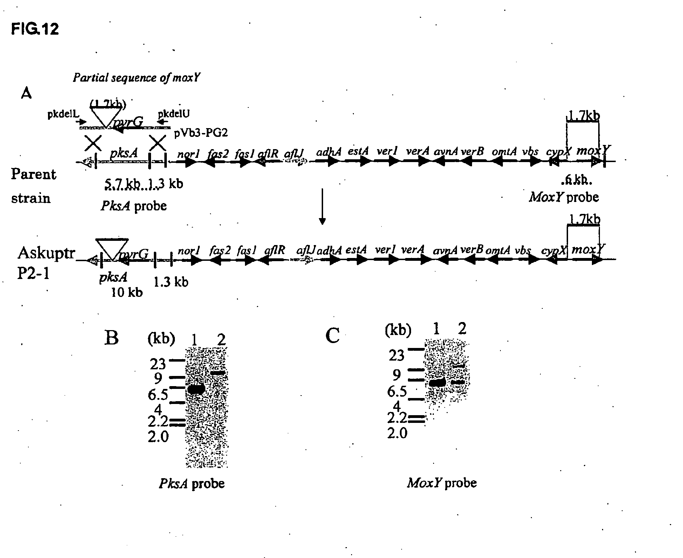

A 3 kb fragment comprising pksA region was amplified by PCR using primers pkdelU and pkdelL (Table 4), and cloned by means of TOPO-TA cloning kit (invitrogen Co.). The resulting plasmid was digested with Smal and subjected to dephosphorylation and ligated with a 1.7kb fragment (SEQ ID No. 7). The 1.7kb fragment was obtained by PCR amplification using primers vbdelU and vbdelL to give a 2.1kb fragment comprising moxY, followed by digestion with Hincll. The resulting plasmid was digested with Hpal, subjected to dephosphorylation and ligated with a fragment obtained by digestion of a 2.7kb fragment comprising pyrG marker, which had been amplified using primers of pyrGUst and pyrGLst, so as to give a vector (pVb3-PG2) for a deletion of a full length of the cluster of Aflatoxin-biosynthesis genes (Fig. 12 A).

**Table 4**

| Primers | Sequence (5' to 3') |
|---|---|
| pk-delU | ACGGCTTCGGCGTTACCTCGTTCAACC |
| pk-delL | TGGTGCATGGCGATGTGGTAGTTCTGG |
| vb-delU | GGCCGCCATTGAAGGTCAATTTGCACA |
| vb-delL | GGCGGTGCGCTGGCGGAAAAACGCAGA |
| pyrGUst | CCCCAGGCCTGTCAGATATGTTCAACGA |
| pyrGLst | CGAGGCCTTTACCAAGGTATCGCGAGCGTA |
| pkU | GGTGCACTGCGTGTGGTCCTGCAGACTACA |
| pkL | GTCGCCGCCGGATCTCATTGCAAAAGCTCT |
| moxU | GCACACCGGACGGAATTGAAATATCTC |
| moxL | GGCGGTGCGCTGGCGGAAAAACGCAGA |

### Southern hybridization:

Southern hybridization was carried out in accordance with a usual method using a membrane filter of Hybond-N+ (Amersham Pharmacia). The detection was done with DIG Luminescent Detection Kit (Roche) in accordance with a manufacturer's instruction. Probes for pksA and moxY were prepared with PCR DIG Probe Synthesis kit (Roche) using primers of pkU and pkL, and moxU and moxL, respectively.

### Production of a strain having a deleted region in chromosome :

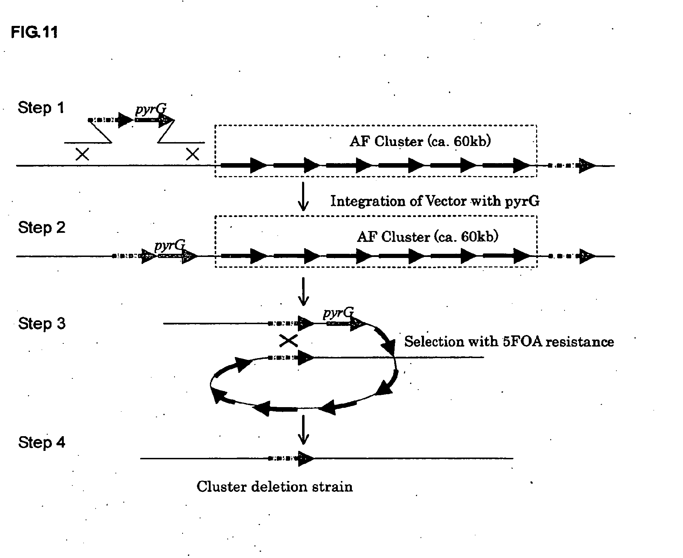

A parent strain of *Aspergillus sojae* ASKUPTR8 was transformed with a 8kb fragment that had been amplified by PCR using the primers of pkdelU and pkdelL and the vector pVb3-PG2 as a template (Fig. 11, Fig.12 (A)). The genomic DNA was extracted from the resulting six transformants, and subjected to southern hybridization to confirm the integration of said vector. As a result, a pksA probe showed the shift of two bands at 5.7kb and 1.3 kb in the parent strain (Fig. 12 (B), lane 1) to 10 kb and 1.3 kb in the vector-integrated strain (Fig. 12 (B), lane 2), respectively. Although only one band was shown at 6kb with a moxY probe in the parent strain (Fig. 12 (C), lane 1), two bands were detected at 10kb in addition to 6kb in the vector-integrated strain (Fig. 12 (C), lane 2). These results supported the integration of the vector into pksA site and the production of a vector-integrated strain, AskuptrP2-1 strain. Such integration of the vector was observed in all of the above six transformants. AskuptrP2-1 strain derived from *Aspergillus sojae* was deposited as an International Deposit under Budapest Treaty on February 1, 2006 with Accession No. FERM BP-10498 at the International Patent Organism Depository of National Institute of Advanced Industrial Science and Technology at AIST Tsukuba Central 6, 1-1, Higashi 1- chome Tsukuba-shi, Ibaraki-ken 305-8566 Japan.

Conidium was recovered from the vector-integrated strain (AskuptrP2-1), inoculated on CZ plate containing 5FOA and cultured for 10 days at 30°C to select a 5FOA-resistant strain in order to obtain a strain wherein the full length of the cluster of Aflatoxin genes was deleted. The frequency of appearance of the 5FOA-resistant strain was 1/1x10⁵.

Ten 5FOA-resistant strains were selected and their extracted DNA was subjected to southern hybridization. A lane 1 of Fgi.14 (B) corresponds to the parent strain, and lanes 2-11 correspond to the 5FOA-resistant strains (AskuptrP2-1-F1 - AskuptrP2-1-F10). While the pksA probe showed two bands at 5.7kb and 1.3 kb in the parent strain after Bglll digestion (Fig. 14 (B), Lane 1), these bands shifted to 7.5kb in nine strains out of the ten 5FOA-resistant strains (Fig. 14 (B), Lane 2, Lanes 4-11). As two bands at 10kb and 1.3kb were observed in one strain (Fig.14(B), Lane 3), it seemed that the cluster had not been excised and the vector remained in the chromosome of this strain. While the moxY probe showed a 6kb band in the parent strain (Fig. 14 (C), Lane 1), the band was shifted to 7.5kb in the nine strains out of the ten 5FOA-resistant strains (Fig. 14 (C), Lane 2, Lanes 4-11). These results conformed with a pattern for excision of the full length of the Aflatoxin cluster, confirming that the full length of the cluster of Aflatoxin-biosynthesis genes was deleted in these strains (AskuptrP2-1-F1, AskuptrP2-1=F3 -AskuptrP2-1-F10) (Fig. 14 (A), lower). As two bands were observed in the other strain (AskuptrP2-1-F2), it was confirmed that the vector remained in the strain, causing some kind of mutation at pyrG site to inactivate it. AskuptrP2-1-F1 strain derived from *Aspergillus sojae* was deposited as an International Deposit under Budapest Treaty on February 1, 2006 with Accession No. FERM BP-10499 at the International Patent Organism Depository of National Institute of Advanced Industrial Science and Technology at AIST Tsukuba Central 6, 1-1, Higashi 1- chome Tsukuba-shi, Ibaraki-ken 305-8566 Japan.

The above results confirmed that an inner region of the Aflatoxin cluster (about 55 kb) had been excised the nine strains out of the ten 5FOA-resistant strains, and that an Aflatoxin-cluster deletion strain could be easily produced. Accordingly, it was demonstrated that the strain having a large chromosomal region, which had been very difficult with respect to *Aspergillus* in the prior arts, could be efficiently obtained by the present invention. As a pyrG marker is deleted in the thus obtained mutant stain of the present invention, which can be repeatedly modified using the pyrG marker. Furthermore, as said strain does not contain any insertion such as a foreign DNA, it can be used for the production of a mutant strain that can be used for the production of foods without any problem, which is considered very advantageous from an industrial point of view.

As the cluster of the genes of toxic substances such as Aflatoxin has been deleted in the strain having a deleted region in chromosome produced by the present method, said strain is very preferable from the aspect of safety. As a result, it will make it possible to genetically modify the *Aspergillus* in chromosome level more efficiently, which has been very difficult in the prior arts.

### SEQUENCE LISTING

<110> NODA INSTITUTE FOR SCIENTIFIC RESEARCH
<120> Method for the prodcution of chromosomal region-deficient strain
<130> AB06007
<160> 7
<210> 1
   <211> 2249
   <212> DNA
   <213> Aspergillus sojae
<220>
   <223> Aspergillus sojae Ku70
<400> 1
<210> 2
   <211> 2707
   <212> DNA
   <213> Aspergillus sojae
<220>
   <223> Aspergillus sojae Ku80 Long
<400> 2
<210> 3
   <211> 2666
   <212> DNA
   <213> Aspergillus sojae
<220>
   <223> Aspergillus sojae Ku80 Short
<400> 3
<210> 4
   <211> 2249
   <212> DNA
   <213> Aspergillus oryzae
<220>
   <223> Aspergillus oryzae Ku70
<400> 4
<210> 5
   <211> 2703
   <212> DNA
   <213> Aspergillus oryzae
<220>
   <223> Aspergillus oryzae Ku80 Long
<400> 5
<210> 6
   <211> 2662
   <212> DNA
   <213> Aspergillus oryzae
<220>
   <223> Aspergillus oryzae Ku80 Short
<400>
<210> 7
   <211> 1746
   <212> DNA
   <213> Aspergillus sojae ASKUPTR8 strain
<400> 7
   aacctcctcg gggccgatga gcacaatagg tccatacttc tcatggaggg attgcacata    60
   ataaatgcgg ttgttggcaa aagatgccca ggcaagccga gcactcgtca ggcgtgcata   120
   ccagggacca gggaccttac gaaggggact gaaatatgcg gtttgaagaa tctaggcgaa   180
   gagttaaagg taccattctg ccatttgtcc atccagaagc tgcggaacta tgacttacct   240
   tgacaatctt gctcacaatc caggcaccgc ccacagcgag gaaccaccag gtgagcggaa   300
   catgctcgat ggcccgaatg agctctcttg acgcagtgtt ggtcatagtt tatagaagga   360
   agcggatatg aaacatctaa ccgtgggttc cctggggtca agacgtctaa catatggtga   420
   cgcacaaata gtgccttcgg tacggacacc ggacggaatt gaaatatctc tcgcgcaccg   480
   gaatcttgtt cctggtcctt tctgtcagtt taattacggt cgtgcctagt atatgacgct   540
   atttcattcg gatacgctct acacgtttac attatggggc ataatacggt gttttactcc   600
   atatataatc aaggcaaagc tactataatc atcattgcat ttttacgctc aaatgctccc   660
   tagtcaagca gcgataaggt gacaaactaa gctgcctaat tcttacttaa ctgccctgga   720
   ttctgacgaa tgactaacat gaatatacca tttgtagaca agtattaccg tctcggccat   780
   cggggacttg tcgctcgccg aatgcaagta tttgggaatg gcctcatcgc tctacacaag   840
   ctcaagataa cgctctctat ctcattagga tcacactgct cataacagaa caaaatgtca   900
   aaggtggact actctcagcc tggctcgact gggtatgaaa tccccccaga tctcacctgg   960
   atggaccccg ccaaccggcc gttgcgggtg gtaaccatcg gcacgggcat ctcggggata  1020
   ctgatggcat accagatcca aaaacaatgc cctaatgtcg agcacgtttt gtatgagaaa  1080
   aatgcggacg tgggtgggac ctggttagaa aaccgttacc ccatggccgg ttgcgatgtc  1140
   ccgagccatg cctacaccta cccctttgct ccgaatccag actggccccg atacttctcc  1200
   tatgcgtccg atatctggaa ttacctcgac cgggtatgca aattctttga tcttcgccgt  1260
   tacatggtgt ttcacacgga ggtcgtgggc tgctactgga acgaagaccg cggagaatgg  1320
   actgtccgac ttcgacagca cgctagcggc agtgagcccc gagagtttga agaccattgt  1380
   cacgtcttgg ttcacgcgtc gggggtattc aataatcctc agtggccgca aatccccggc  1440
   ctccatgacc ggttccaagg ccgtgtgctc cataccgcac gatggcccga cgactaccaa  1500
   gagtcacaat ggaagagtga tagggtggca gtcattggct ccggggcatc atccatccaa  1560
   acggtgcccg gcatgcagcc cacggtgaag catctcgatg tctttgtgcg gacgggcgta  1620
   tggttcggag tgctagcggg caacactggc agccagacca aggaatactc gcccaccgag  1680
   cgcgacgaat tccggcgcaa tccagcagcg cttgtggccc acgcaaaggc catcgaggac  1740
   caggtc                                                             1746

## Claims

1. A method for the production of a strain having a deleted region in chromosome using a transformant derived from *Aspergillus sojae* or *Aspergillus oryzae* and having an increased frequency of homologous recombination due to disruption of ku70 and/or ku80, comprising transforming said transformant so as to include a homologous region in both ends of a chromosomal region to be deleted, and deleting the chromosomal region by means of homologous recombination between said homologous region, wherein the homologous region is a DNA sequence originally existing in the same chromosome as that of the chromosomal region to be deleted and the homologous region consists of a DNA sequence comprising a gene or its part which is located at the most peripheral site in either end of the chromosomal region to be deleted, wherein the method further comprises amplifying a DNA sequence comprising a gene or its part which is located at the most peripheral site in one end of the chromosomal region to be deleted, and integrating the amplified sequence into a gene located at the most peripheral site in the other end of said chromosomal region as a homologous region by homologous recombination so that the homologous regions will be included in both ends of said chromosomal region.

2. A method according to Claim 1, wherein the chromosomal region constitutes a cluster of genes involved in biosynthesis of a toxic substance.

3. A method according to Claim 2, wherein the toxic substance is Aflatoxin.

4. A method according to Claim 1, wherein the homologous region is a DNA sequence comprising moxY gene, and is integrated into pksA gene.

5. A method according to Claim 4, wherein the homologous region has a DNA sequence represented by SEQ ID No.7.

6. A method according to any one of Claims 1-6, wherein a marker gene used for negative selection is comprised in the chromosomal region to be deleted.

7. A method according to Claim 6, wherein the marker gene used for negative selection is selected from pyrG, sC or niaD.

## Patentansprüche

1. Verfahren zur Produktion eines Stammes, der unter Verwendung einer von Aspergillus sojae oder Aspergillus oryzae abgeleiteten Transformante eine deletierte Region im Chromosom und aufgrund der Unterbrechung von ku70 und/oder ku80 eine erhöhte Frequenz der homologen Rekombination aufweist, welches das Transformieren der Transformante, so dass die homologe Region in beiden Enden einer zu deletierenden chromosomalen Region eingebaut wird, und das Deletieren der chromosomalen Region mittels homologer Rekombination zwischen den homologen Regionen umfasst, wobei die homologe Region eine DNA-Sequenz ist, die ursprünglich in demselben Chromosom wie diejenige der zu deletierenden chromosomalen Region vorhanden war und die homologe Region aus einer DNA-Sequenz besteht, die ein an der äußersten peripheren Stelle an einem Ende der zu deletierenden chromosomalen Region befindliches Gen oder einen Teil davon umfasst, wobei das Verfahren außerdem das Amplifizieren einer DNA-Sequenz, die ein an der äußersten peripheren Stelle in einem Ende der zu deletierenden chromosomalen Region befindliches Gen oder einen Teil davon umfasst, und das Integrieren der amplifizierten Sequenz in ein an der äußersten peripheren Stelle an dem anderen Ende der chromosomalen Region als eine homologe Region befindliches Gen durch homologe Rekombination umfasst, so dass die homologen Regionen in beide Enden der chromosomalen Region eingebaut werden.

2. Verfahren nach Anspruch 1, wobei die chromosomale Region ein Cluster von Genen ist, die an der Biosynthese einer toxischen Substanz beteiligt sind.

3. Verfahren nach Anspruch 2, wobei die toxische Substanz Aflatoxin ist.

4. Verfahren nach Anspruch 1, wobei die homologe Region eine das moxY-Gen umfassende DNA-Sequenz ist und in das pksa-Gen integriert wird.

5. Verfahren nach Anspruch 4, wobei die homologe Region eine DNA-Sequenz der Sequenz ID No. 7 darstellt.

6. Verfahren nach einem der Ansprüche 1 bis 6, wobei ein für die negative Selektion verwendetes Markergen in der zu deletierenden chromosomalen Region enthalten ist.

7. Verfahren nach Anspruch 6, wobei das für die negative Selektion verwendete Markergen unter pyrG, sC oder niaD ausgewählt ist.

## Revendications

1. Procédé pour la production d'une souche ayant une région délétée dans un chromosome utilisant un transformant dérivé de *Aspergillus sojae* ou *Aspergillus oryzae* et ayant une fréquence accrue de recombinaison homologue due à l'interruption de ku70 et/ou ku80, comprenant la transformation dudit transformant de manière à inclure une région homologue dans les deux extrémités d'une région chromosomique à déléter, et à la délétion de la région chromosomique par recombinaison homologue entre ladite région homologue, où la région homologue est une séquence d'ADN existant originellement dans le même chromosome que celui de la région chromosomique à déléter et la région homologue consiste en une séquence d'ADN comprenant un gène ou sa partie qui est située au site le plus périphérique dans les deux extrémités de la région chromosomique à déléter, où le procédé comprend en outre l'amplification d'une séquence d'ADN comprenant un gène ou sa partie qui est située au site le plus périphérique dans une extrémité de la région chromosomique à déléter, et l'intégration de la séquence amplifiée dans un gène situé au site le plus périphérique dans l'autre extrémité de ladite région chromosomique sous forme d'une région homologue par recombinaison homologue de sorte que les régions homologues seront incluses dans les deux extrémités de ladite région chromosomique.

2. Procédé selon la revendication 1, où la région chromosomique constitue un cluster de gènes impliqués dans la biosynthèse d'une substance toxique.

3. Procédé selon la revendication 2, où la substance toxique est l'aflatoxine.

4. Procédé selon la revendication 1, où la région homologue est une séquence d'ADN comprenant le gène moxY, et est intégrée dans le gène pksA.

5. Procédé selon la revendication 4, où la région homologue a une séquence d'ADN représentée par SEQ ID No : 7.

6. Procédé selon l'une quelconque des revendications 1-6, où un gène marqueur utilisé pour la sélection négative est compris dans la région chromosomique à déléter.

7. Procédé selon la revendication 6, où le gène marqueur utilisé pour la sélection négative est choisi parmi pyrG, sC et niaD.
